(19) Europäisches Patentamt
European Patent Office
Office européen des brevets

(11) Veröffentlichungsnummer: **0 394 792 A1**

(12) **EUROPÄISCHE PATENTANMELDUNG**

(21) Anmeldenummer: **90107142.3**

(22) Anmeldetag: **13.04.90**

(51) Int. Cl.5: **C07D 295/185, C09J 4/02, A61K 6/00, C08L 33/14, C08L 33/26**

(30) Priorität: **27.04.89 DE 3913940**

(43) Veröffentlichungstag der Anmeldung:
**31.10.90 Patentblatt 90/44**

(84) Benannte Vertragsstaaten:
**AT BE CH DE DK ES FR GB GR IT LI LU NL SE**

(71) Anmelder: **BAYER AG**

**D-5090 Leverkusen 1 Bayerwerk(DE)**

(72) Erfinder: **Müller. Michael, Dr.**
**Richard-Zanders-Strasse 34**
**D-5060 Bergisch - Gladbach 2(DE)**
Erfinder: **Podszun, Wolfgang, Dr.**
**Roggendorfstrasse 55**
**D-5000 Koeln 80(DE)**

(54) **Neue, Formylpiperazingruppen enthaltende (Meth)acrylsäurederivate.**

(57) Die Erfindung betrifft neue Formylpiperazingruppen enthaltende (Meth)acrylsäureester und (Meth)-acrylsäureamide (I) der allgemeinen Formel (I)

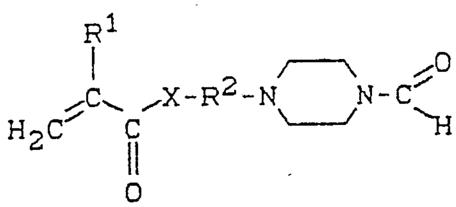

(I),

in der $R^1$, $R^2$ und X die in der Beschreibung angegebene Bedeutung haben und Zubereitungen (II) zur Verwendung als Adhäsivkomponente zur Behandlung kollagenhaltiger Materialien, die diese Verbindungen enthalten, Verfahren zur Herstellung und die Verwendung der Zubereitungen.

EP 0 394 792 A1

# Neue, Formylpiperazingruppen enthaltende (Meth)acrylsäurederivate

Die Erfindung betrifft neue Formylpiperazingruppen enthaltende (Meth)acrylsäurederivate der Formel (I) und Zubereitungen zur Verwendung als Adhäsivkomponente zur Behandlung kollagenhaltiger Materialien, die diese Verbindungen enthalten, sowie Verfahren zur Herstellung und die Verwendung der Zubereitungen.

Die neuen Formylpiperazingruppen enthaltenden (Meth)acrylsäureester und (Meth)acrylsäureamide entsprechen der Formel (I)

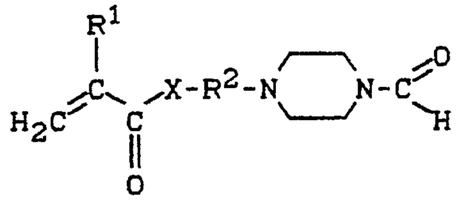

(I),

in der

$R^1$ Wasserstoff oder Methyl bedeutet,

$R^2$ ein zweiwertiger aliphatischer oder cycloaliphatischer Rest bedeutet und

X die Bedeutung von -O- oder -NH- hat.

Die Erfindung betrifft außerdem Zubereitungen zur Verwendung als Adhäsivkomponente zur Behandlung kollagenhaltiger Materialien, die Verbindungen (I) und gegebenenfalls Initiatoren und Lösungsmittel enthalten, Verfahren zur Herstellung und die Verwendung der Zubereitungen.

Kollagenhaltige Materialien sind Gerüsteiweißkörper und Hauptbestandteile der menschlichen und tierischen interzellularen Stützsubstanzen wie Knorpel- und Knochengewebe, Haut und Zahnbein (Dentin). Im Rahmen der vorliegenden Erfindung werden die Adhäsivkomponenten bevorzugt zur Behandlung von Dentin im Zusammenhang mit Zahnreparaturen verwendet.

Besonders im Dentalbereich werden härtende, polymere Materialien als Füllmaterialien bei Zahnreparaturen verwendet. Als härtende, polymere Materialien werden im allgemeinen Füllungen auf Acrylatbasis bevorzugt. Diese polymeren Füllungen haben jedoch den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus, beachtliche Mengen an frischem Dentin zu entfernen.

Nach einer anderen Methode ätzt man das Dentin und die Schmelzoberfläche mit Säuren, wie z.B. Phosphorsäure, an, und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa).

In J. Dent, Res. 57, 500-505 (1978), werden aldehydgruppenhaltige Methacrylate der isomeren Hydroxybenzaldehyde beschrieben, die als Grundierungsmittel für Füllungen im Dentalbereich verwendet werden können. Die Bindung zwischen Dentin und Füllmasse bleibt jedoch auch nach einer solchen Grundierung unbefriedigend.

In Scand. J. Dent. Res. 92, 480-483 (1984) und J. Dent. Res. 63, 1087-1089 (1984) werden Grundierungsmittel aus wäßrigem Formaldehyd oder Glutaraldehyd und β-Hydroxyethylmethacrylat (HEMA) beschrieben.

Außerdem sind in der EP-A 0 141 324 Zusammensetzungen aus einem Aldehyd und einem olefinisch ungesättigten Monomer mit aktivem Wasserstoff beschrieben, die eine gute Anbindung an Dentin aufweisen.

Die neuen Zubereitungen auf Basis von Formylpiperazingruppen enthaltenden (Meth)acrylsäureestern bzw. (Meth)acrylsäureamiden (I) bewirken eine starke Verbundhaftung von Materialien, die am Kollagen befestigt werden sollen, z.B. eine Verbundhaftung von Zahnfüllungsmaterial in einer Kavität am Zahn.

Formamidgruppen enthaltende (Meth)acrylsäurealkylester sind aus der DE-A 2 507 189 bekannt. In der DE-A 2 507 189 wird auch beschrieben, diese Acrylsäureester als Überzüge oder Klebemittel für Papier und Textilien zu verwenden.

Die Verwendung der erfindungsgemäßen N-Formyl-N′-[(meth)acryloyloxyalkyl]piperazine und N-Formyl-N′-[(meth)acryloylaminoalkyl]piperazine (I) als Adhäsivkomponente für kollagenhaltige Materialien war überraschend, weil sie keine reaktiven Gruppen enthalten, die unter milden Bedingungen geeignete chemische

Bindungen zu kollagenhaltigen Materialien aufbauen können.

(Meth)acrylsäureester bzw. (Meth)acrylsäureamide im Rahmen der vorliegenden Erfindung sind die Ester bzw. Amide der Acrylsäure und der Methacrylsäure.

Ein zweiwertiger aliphatischer Rest ($R^2$) bedeutet im allgemeinen einen zweiwertigen, geradkettigen oder verzweigten Kohlenwasserstoffrest mit 1 bis 6, bevorzugt 1 oder 2, Kohlenstoffatomen.

Beispielsweise seien die folgenden zweiwertigen aliphatischen Reste genannt:
Hexandiyl, Pentandiyl, Neopentandinyl, Butandiyl, Dimethylethandiyl, Propandiyl, Ethandiyl.

Ein zweiwertiger cycloaliphatischer Rest ($R^2$) bedeutet im allgemeinen einen cyclischen Kohlenwasserstoffrest mit 4 bis 6 Kohlenstoffatomen. Beispielsweise seien die folgenden zweiwertigen cycloaliphatischen Reste genannt: Cyclobutandiyl, Cyclopentandiyl, Cyclohexandiyl.

Beispielsewise seien die folgenden Formylpiperazingruppen enthaltenden (Meth)acrylsäureester und (Meth)acrylsäureamide genannt:

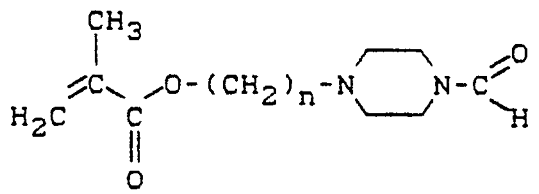

mit n = 3 bis 6

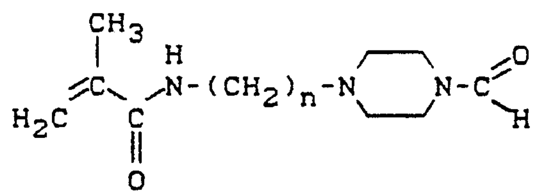

mit n = 3 bis 6

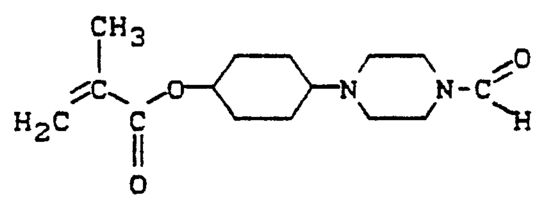

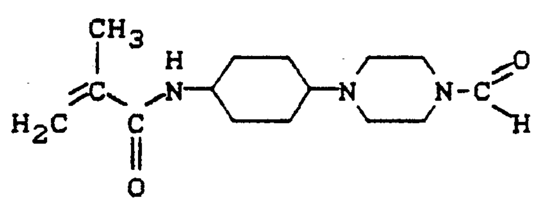

Besonders bevorzugt sind N-Formyl-N′-(methacryloyloxethyl)piperazin und N-Formyl-N′-(methacryloylaminoethyl)piperazin der Formeln

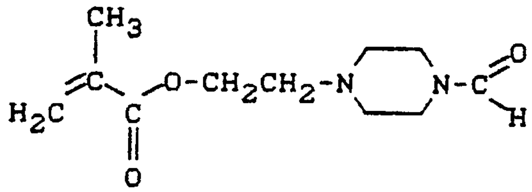

und

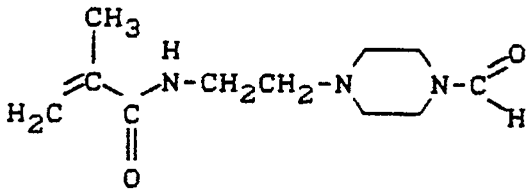

Beispielsweise können die erfindungsgemäßen N-Formyl-N′-[(meth)acryloyloxyalkyl]piperazine durch Umsetzung von N-(Hydroxyalkyl)piperazinen mit Ameisensäureestern und (Meth)acrylsäurechlorid hergestellt werden.

N-Formyl-N′-[(meth)acryloylaminoalkyl]piperazine können überraschenderweise durch entsprechende sukzessive Umsetzung von N-(Aminoalkyl)piperazinen (II) mit Ameisensäurealkylestern (III) und (Meth)-acrylsäurechlorid (IV) hergestellt werden:

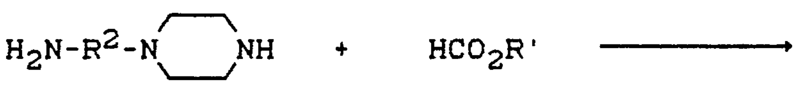

(II) (III)

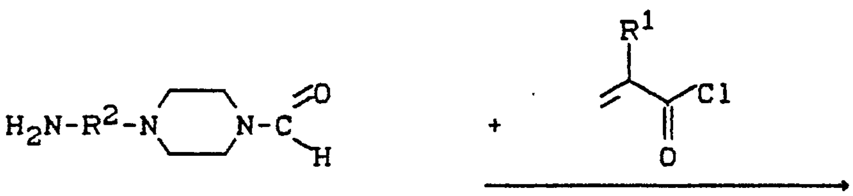

(V) (IV)

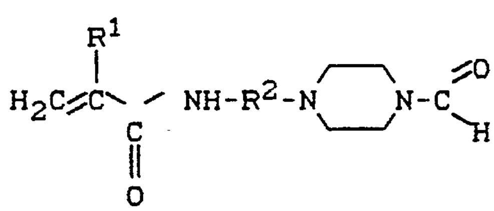

(Ib)

Die Erfindung betrifft außerdem die neuen als Zwischenprodukte erhaltenen N-Formyl-N′-aminoalkylpiperazine (V). Diese Verbindungen konnten bisher nicht erhalten werden, da sich die Aminoalkylpiperazine (II) mit Ameisensäurephenylester (DE-OS 2 209 853) oder auch mit freien Carbonsäuren (US 3 385 858) nicht selektiv am sekundären N-Atom umsetzen ließen.

Es wurde nun gefunden, daß sich diese Formylierung von Aminoalkylpiperazinen (II) mit Ameisensäurealkylestern (III) in hoher Selektivität zu den erfindungsgemäßen N-Formyl-N′-aminoalkylpiperazinen (V) durchführen läßt.

Hierzu werden vorteilhaft Ameisensäureester kurzkettiger Alkohole wie Ameisensäurebutylester oder Ameisensäurepropylester eingesetzt. Besonders bevorzugt sind Ameisensäuremethyl- oder Ameisensäureethylester.

Die erfindungsgemäßen Zwischenstufen (V) sind nicht nur bedeutend für die Darstellung erfindungsgemäßer N-Formyl-N′-[(methyl)acryloylaminoalkyl]-piperazine (Ib), sondern ebenfalls als Zwischenprodukte für Wirkstoffsynthesen einsetzbar.

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitiatoren sind an sich bekannt (Houben-Weyl, Methoden der organischen Chemie, Band E 20, Seite 50 ff, Georg Thieme Verlag Stuttgart 1987). Vorzugsweise handelt es sich um Mono-oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketo-Derivate des Norbornans und substituierter Norbornane, Metallcarbonyle wie Pentacarbonylmangan oder Chinone wie 9,10-Phenanthrenchinon und Naphthochinon. Besonders bevorzugt ist Campherchinon.

Die erfindungsgemäßen Zubereitungen erhalten im allgemeinen 0,01 bis 2 Gew.-Teile, bevorzugt 0,2 bis 0,5 Gew.-Teile des Initiators, bezogen auf 1 Gew.-Teil der N-Formylpiperazingruppen enthaltenden (Meth)-acrylsäureester bzw. (Meth)acrylsäureamide.

Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Die Lösungsmittel im Rahmen der vorliegenden Erfindung sollen die Komponente lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Tetrahydrofuran, Essigsäuremethyl- oder -ethylester genannt.

Im allgemeinen setzt man 10 bis 1000 Gew.-Teile, bevorzugt 50 bis 300 Gew.-Teile des Lösungsmittels, bezogen auf die N-Formylpiperazinderivate ein.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N′,N′-Tetraalkylalkylendiamin, Barbitursäure und Dialkylbarbitursäure.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen eingesetzt.

Als weitere Komponente können die erfindungsgemäßen Zusammensetzungen Carbonylverbindungen enthalten.

Carbonylverbindungen im Rahmen der vorliegenden Erfindung sind Aldehyde und Ketone, die 1 bis 20, bevorzugt 1 bis 10, und besonders bevorzugt 2 bis 6 Kohlenstoffatome, enthalten. Die Carbonylfunktion kann an einem aliphatischen, aromatischen und heterocyclischen Molekülteil gebunden sein.

Als Aldehyde seien aliphatische Mono- oder Dialdehyde genannt. Bevorzugt werden Formaldehyd, Acetaldehyd, Propionaldehyd, 2-Methylpropionaldehyd, Butyraldehyd, Benzaldehyd, Vanillin, Furfural, Anisaldehyd, Salicylaldehyd, Glyoxal, Glutardialdehyd und Phthaldialdehyd. Besonders bevorzugt ist der Glutardialdehyd.

Als Ketone seien besonders aliphatische Mono- und Diketone genannt. Bevorzugt sind Butanon, Aceton, Cyclooctanon, Cycloheptanon, Cyclohexanon, Cyclopentanon, Acetophenon, Benzophenon, 1-Phenyl-2-propanon, 1,3-Diphenyl-2-propanon, Acetylaceton, 1,2-Cyclohexandion, 1,2-Cyclopentandion und Campherchinon. Besonders bevorzugt ist Cyclopentanon.

Im allgemeinen setzt man 1 bis 1000 Gew.-Teile, bevorzugt 5 bis 50 Gew.-Teile der Carbonylverbindungen, bezogen auf die N-Formylpiperazinderivate ein.

Als weitere Komponente können die erfindungsgemäßen Zusammensetzungen (Meth)-acrylsäureester enthalten, die Vernetzungen ausbilden können. (Meth)-acrylsäureester, die Vernetzungen ausbilden können, enthalten im allgemeinen 2 oder mehr polymerisierbare aktive Gruppen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Alkoxy(meth)acrylate und Urethangruppen enthaltende (Meth)acrylate.

Beispielsweise seien (Meth)-acrylsäureester der Formel

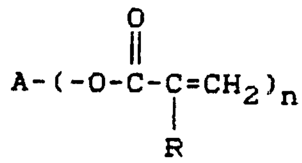

in der
A einen geradkettigen, verzweigten, cyclischen, aliphatischen, aromatischen oder gemischt aliphatischaromatischen Rest mit 2 bis 25 C-Atomen, der durch -O-, NH- oder O-CO-NH-Brücken unterbrochen und mit Hydroxy, Oxy, Carboxy, Amino oder Halogen substituiert sein kann,
bedeutet,
R H oder Methyl bedeutet und
n für eine ganze Zahl von 2 bis 8, vorzugsweise 2 bis 4, steht,
genannt.

Vorzugsweise seien Verbindungen der folgenden Formeln genannt:

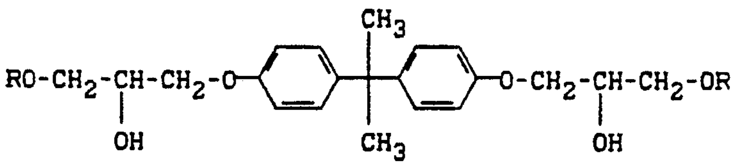

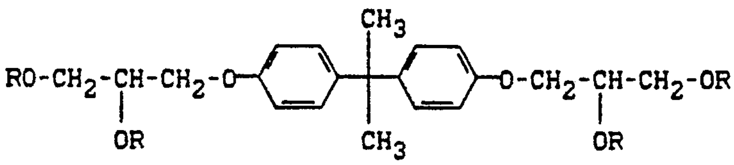

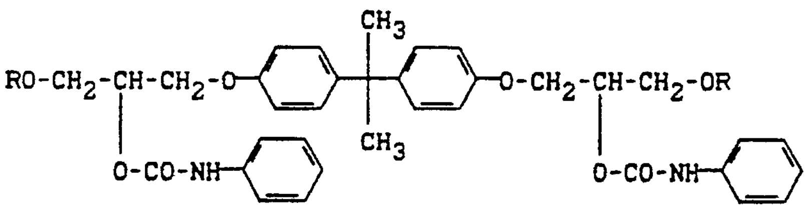

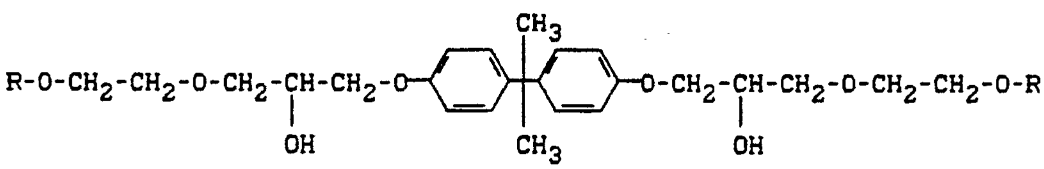

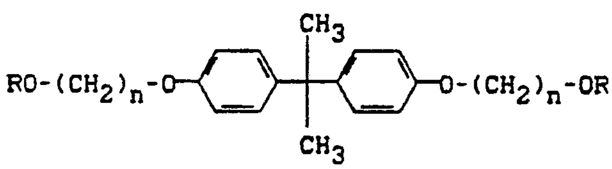

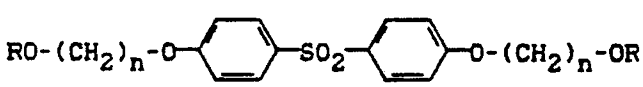

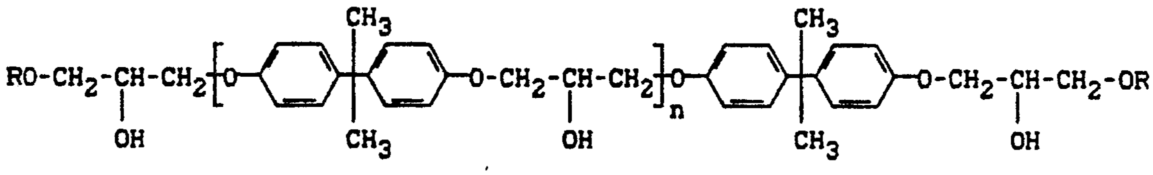

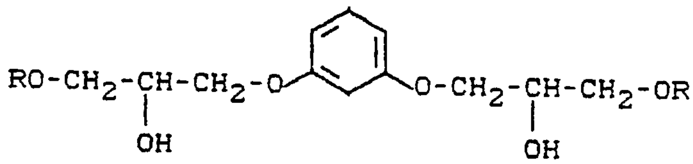

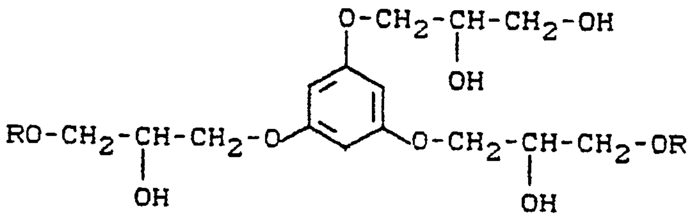

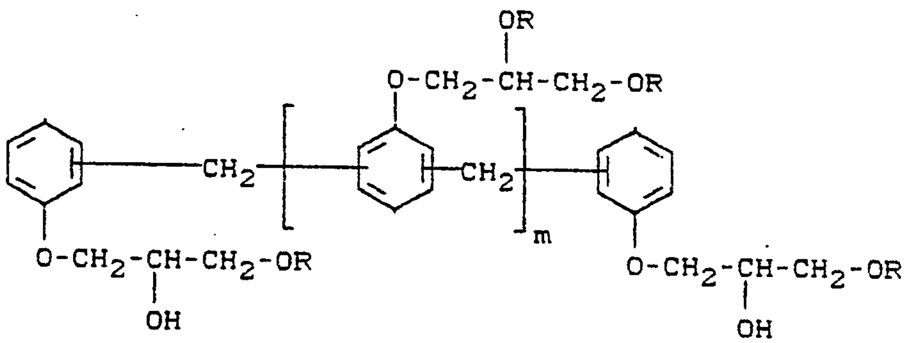

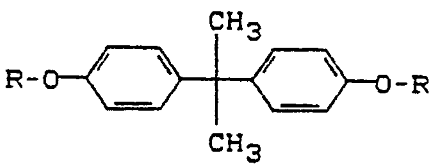

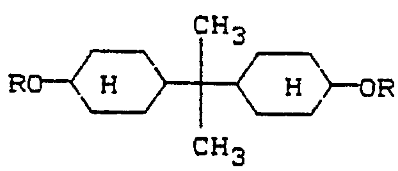

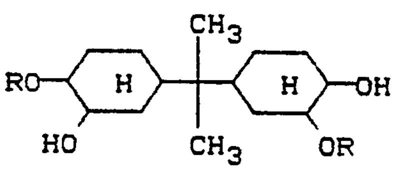

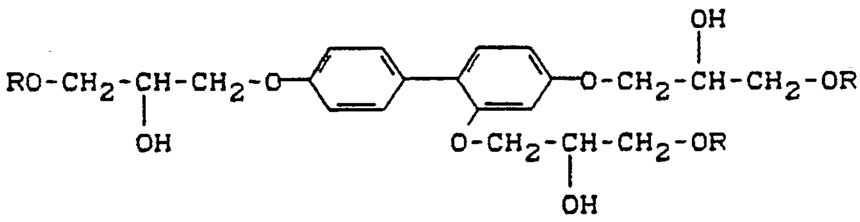

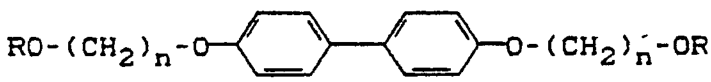

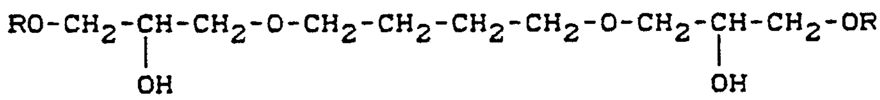

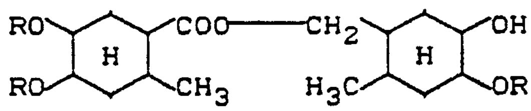

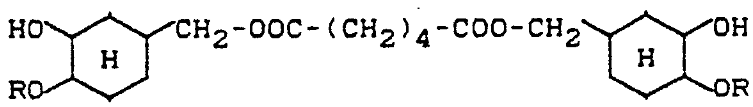

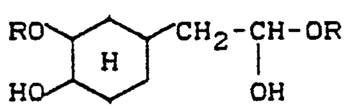

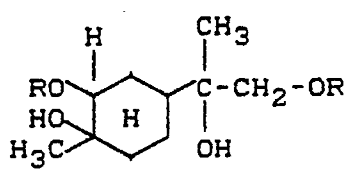

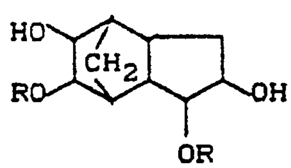

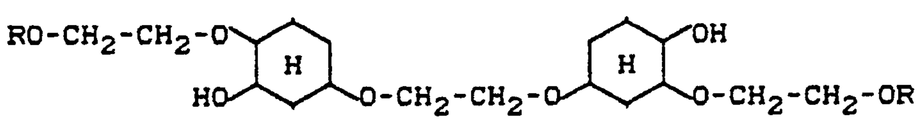

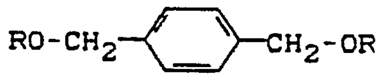

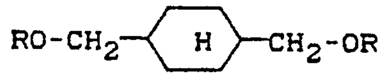

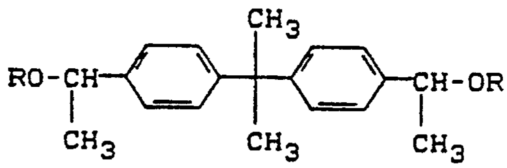

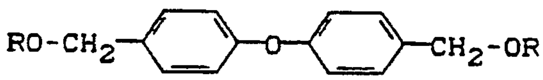

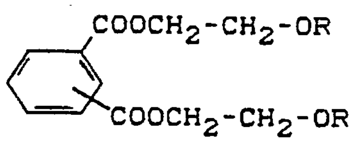

in der ortho-, meta- oder para-Form

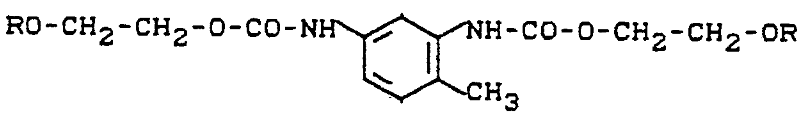

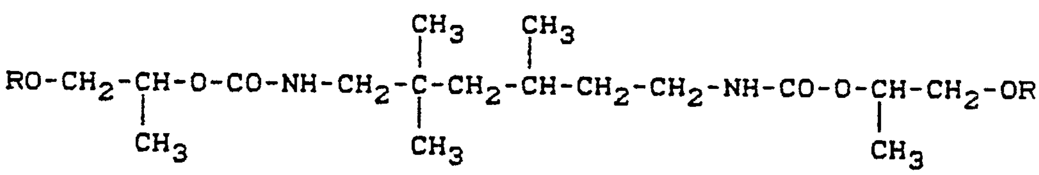

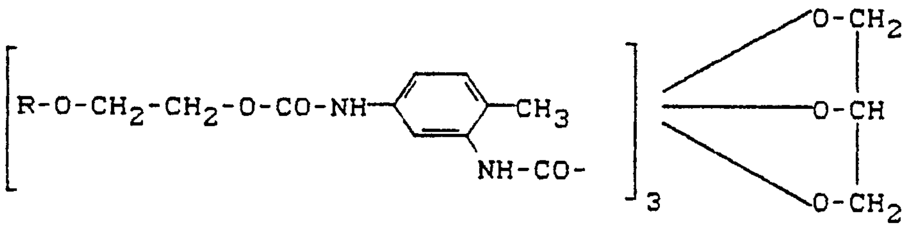

$R-O-(CH_2)_n-O-R$

$R-O-(CH_2CH_2O)_m-R$

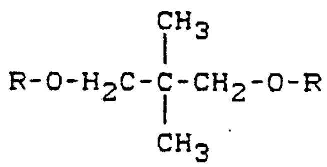

worin

R für 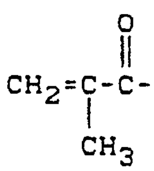 oder 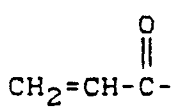

steht,
n eine Zahl von 1 bis 4 und
m eine Zahl von 0 bis 5 bedeutet.

Außerdem seien Derivate des Tricyclodecans (EP-A 00 23 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt:

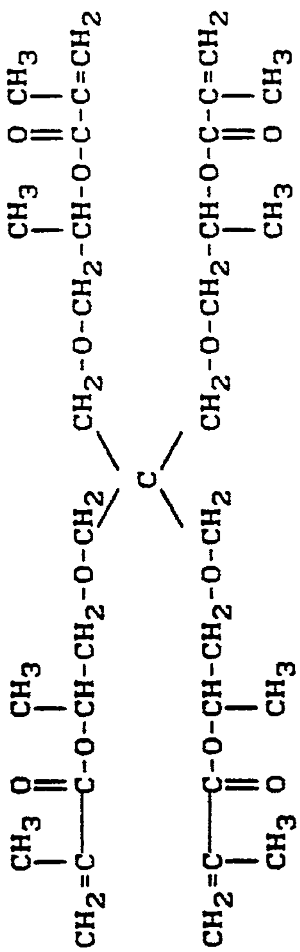

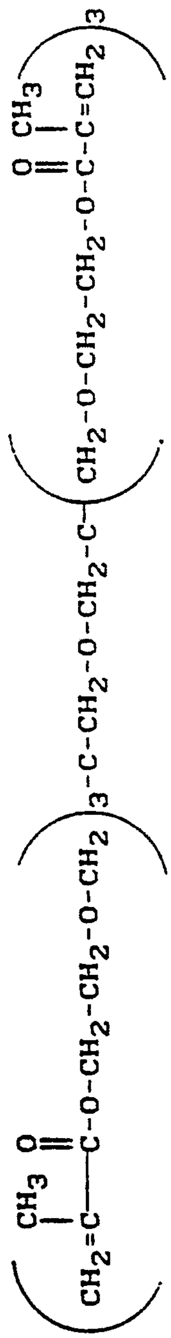

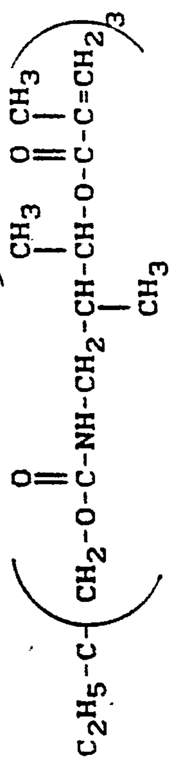

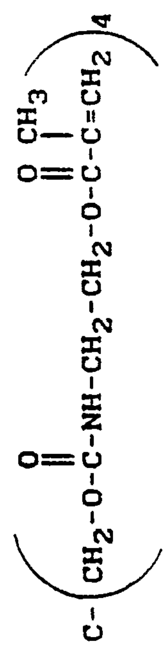

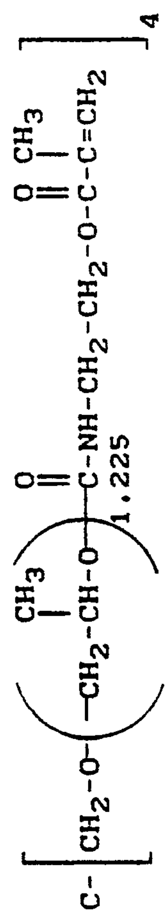

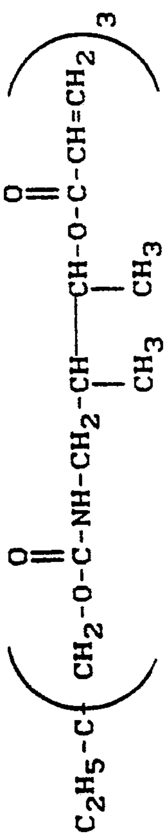

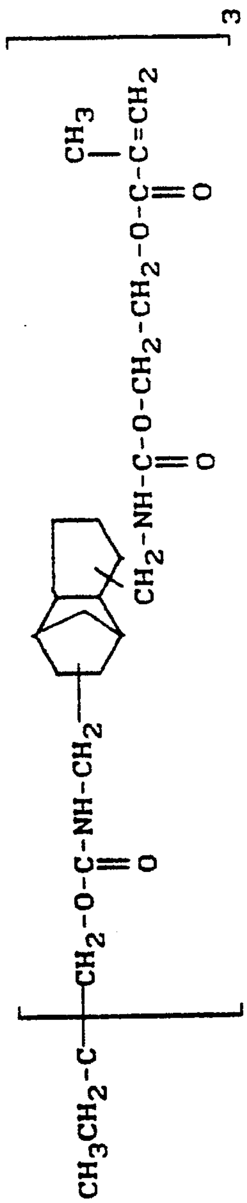

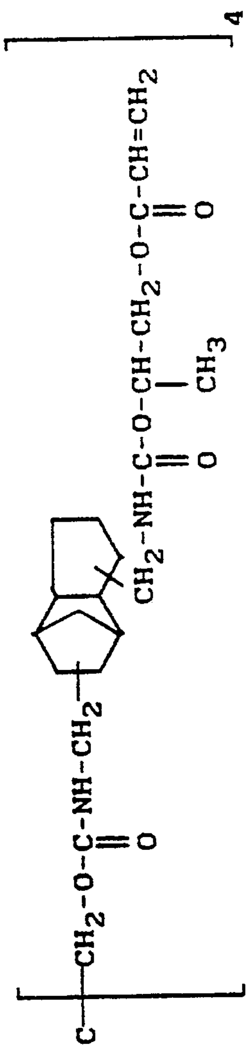

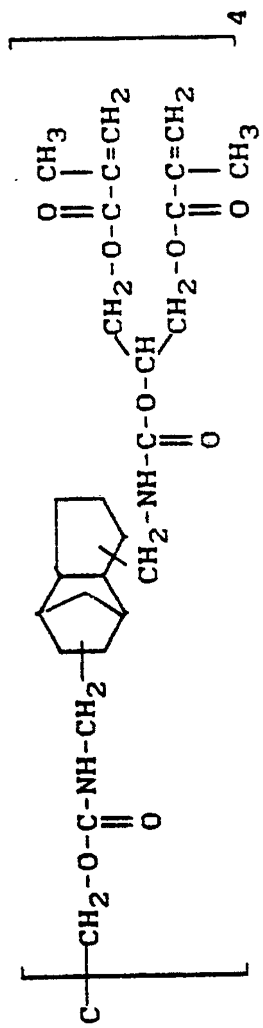

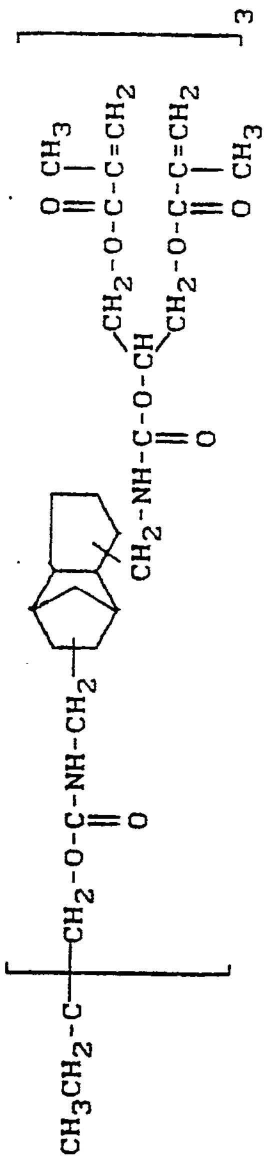

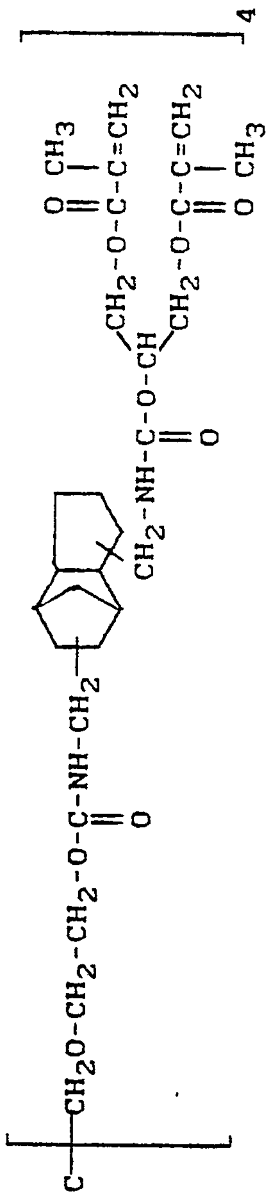

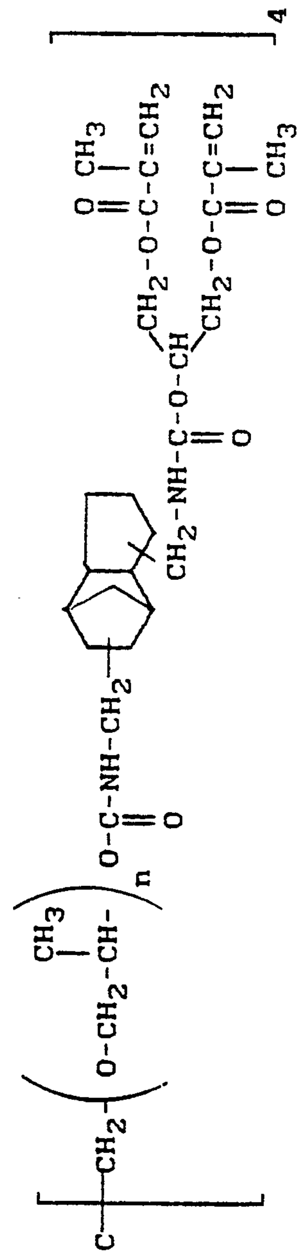

n = 1,225 (statistischer Mittelwert für 4 Ketten)

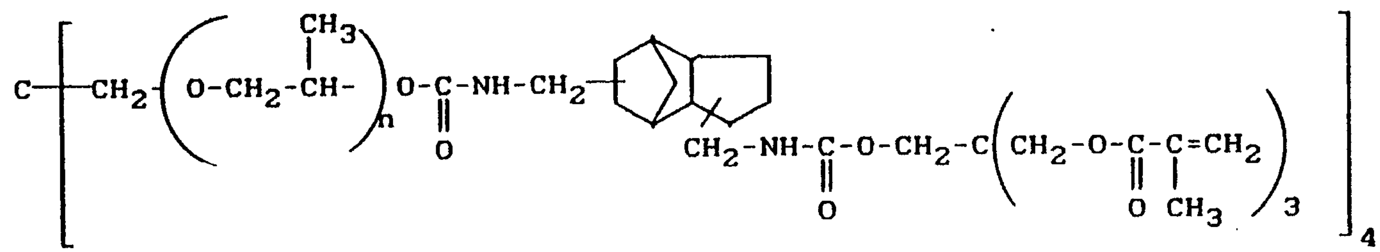

n = 1,225 (Mittelwert)

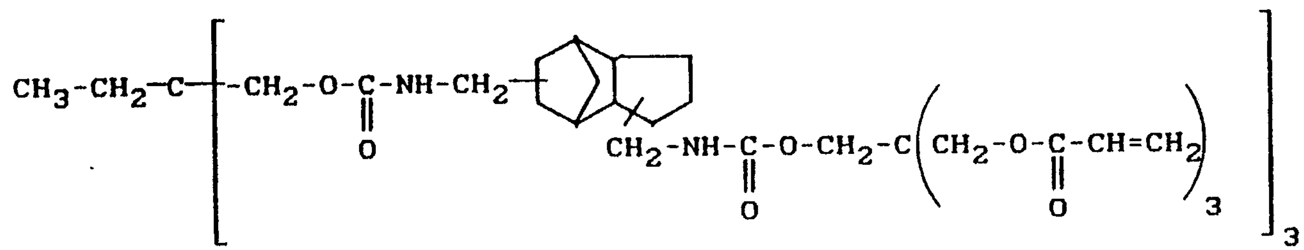

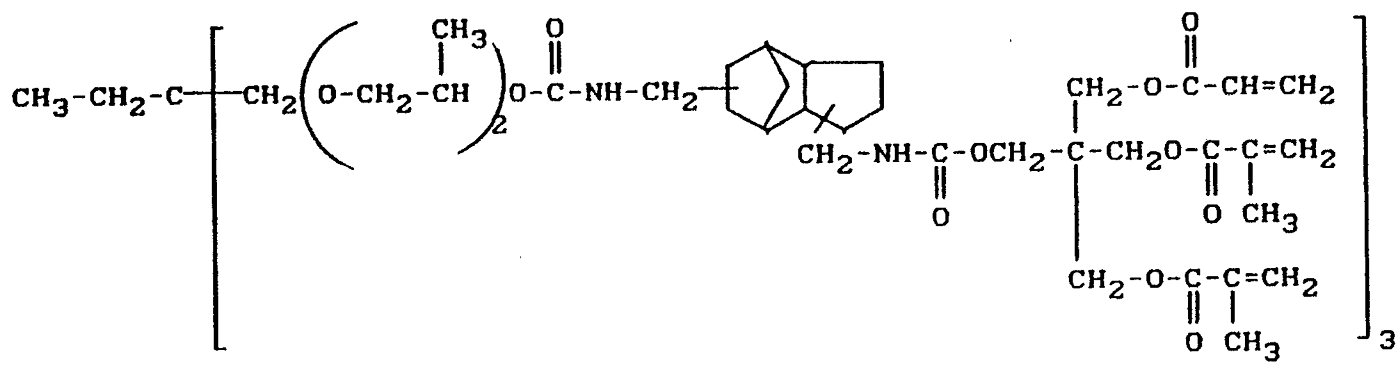

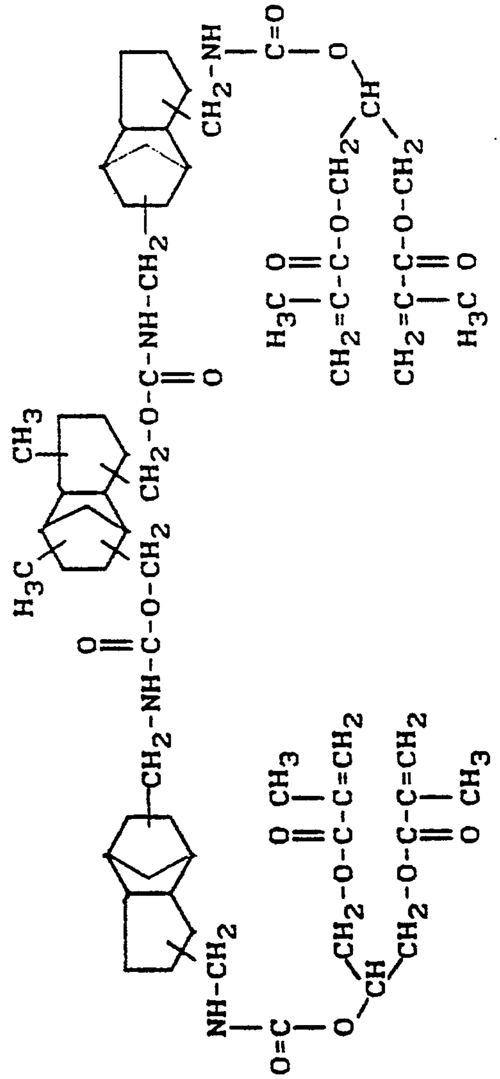

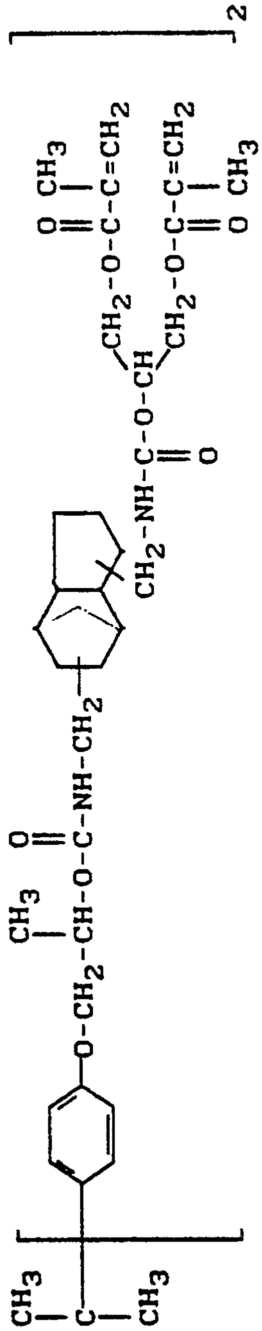

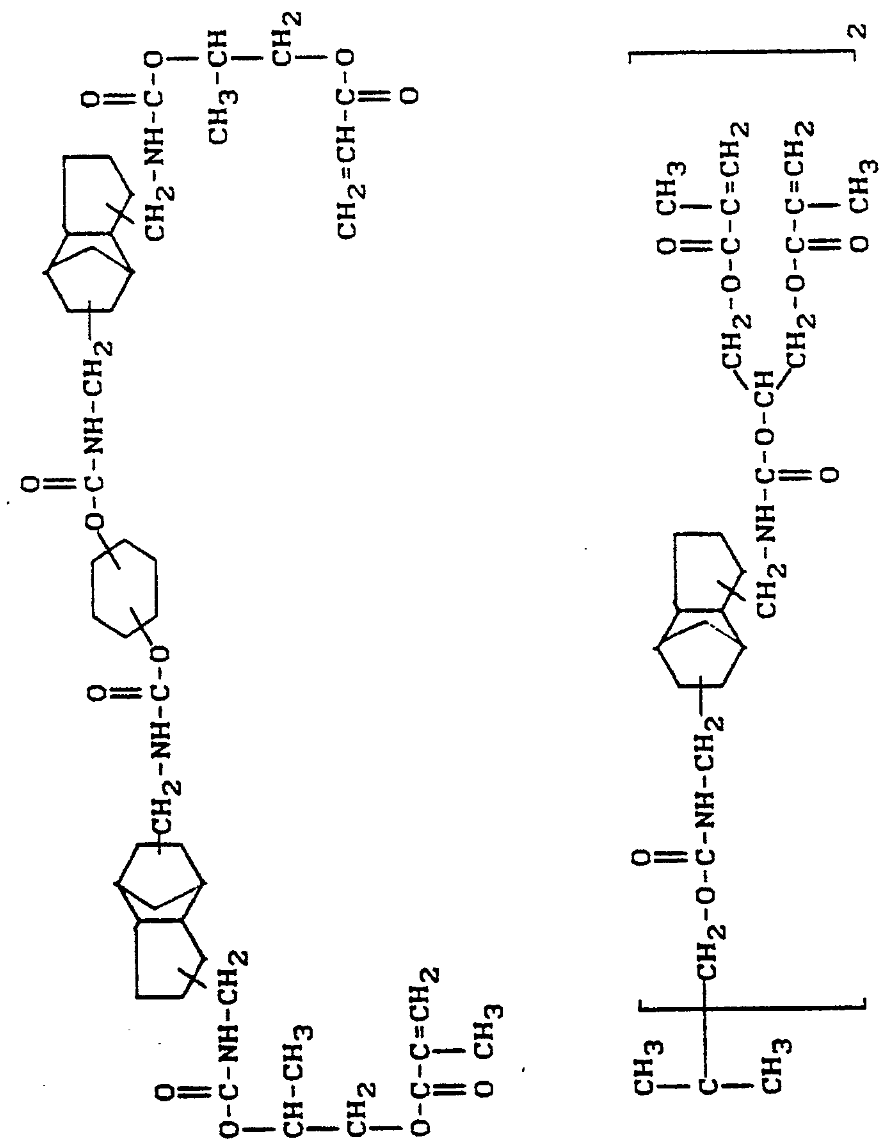

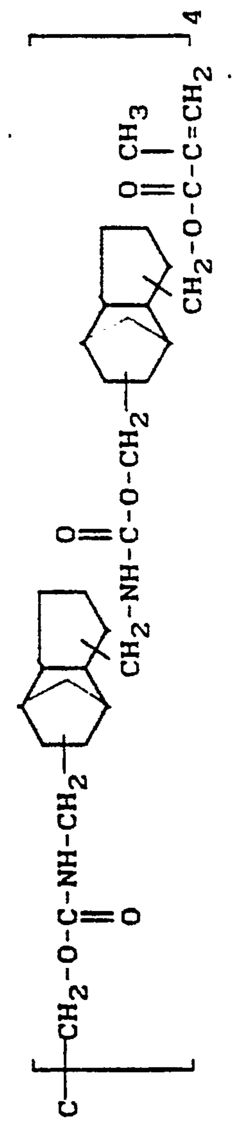

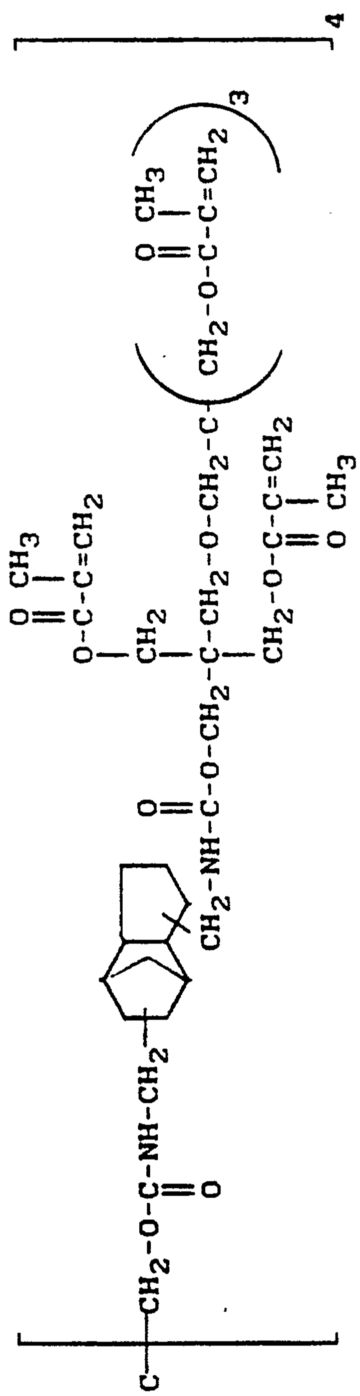

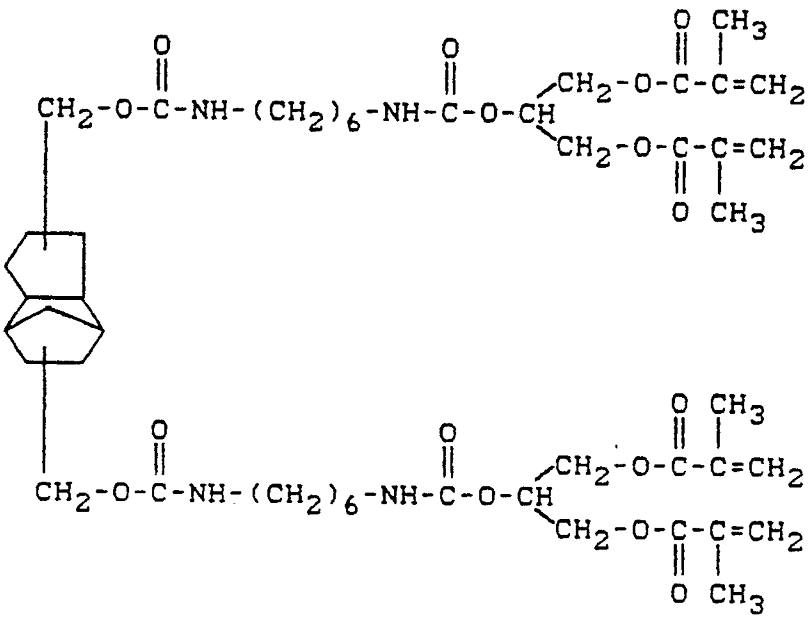

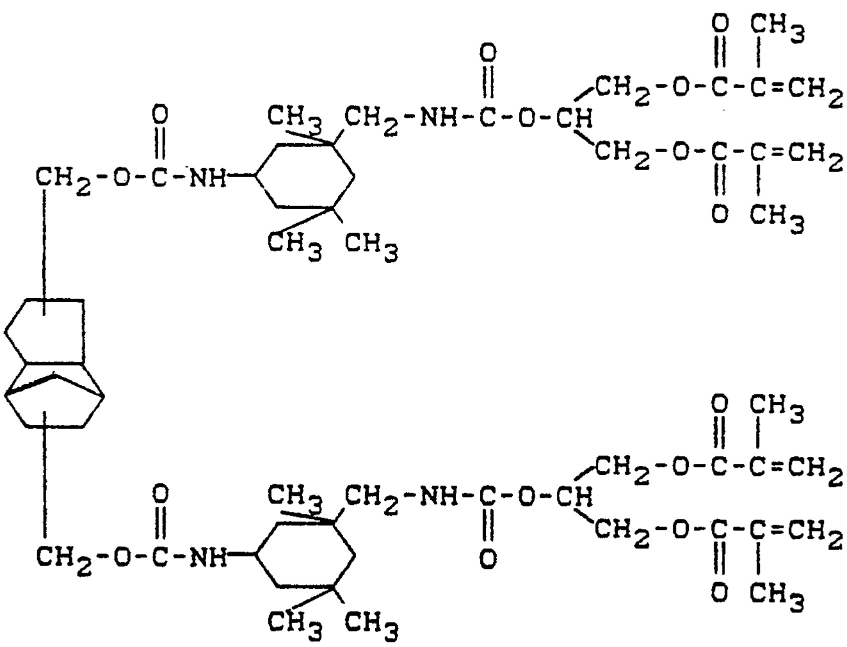

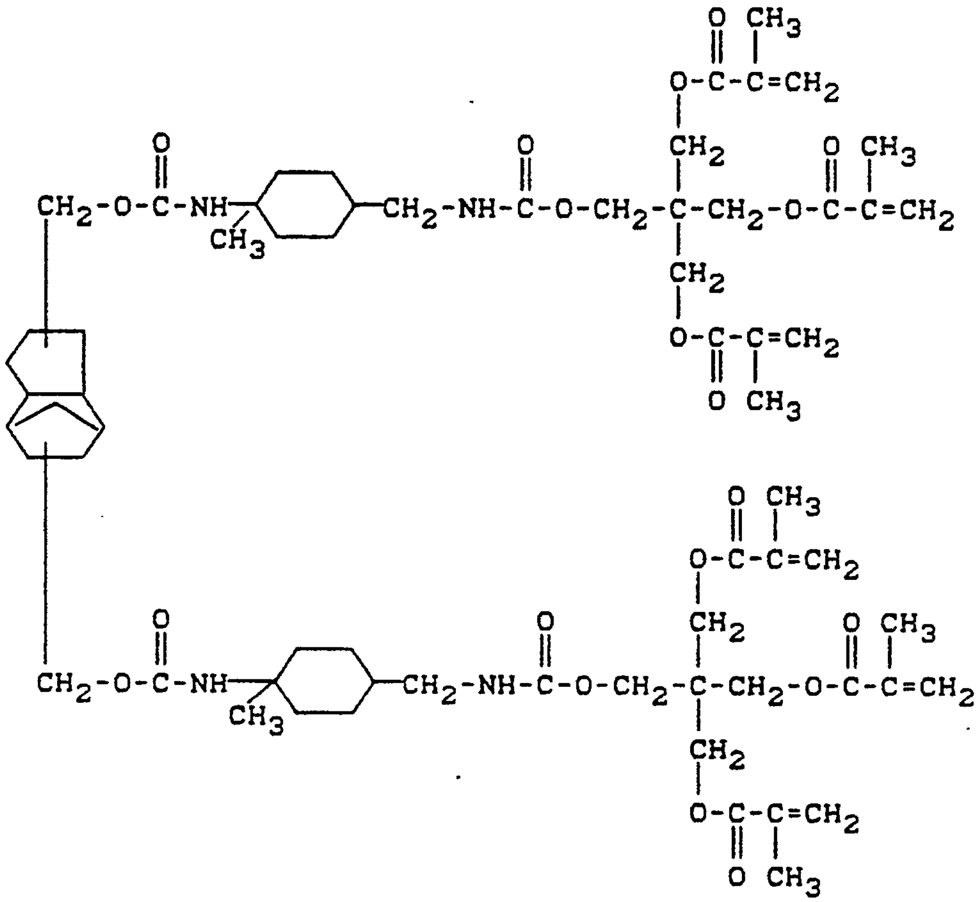

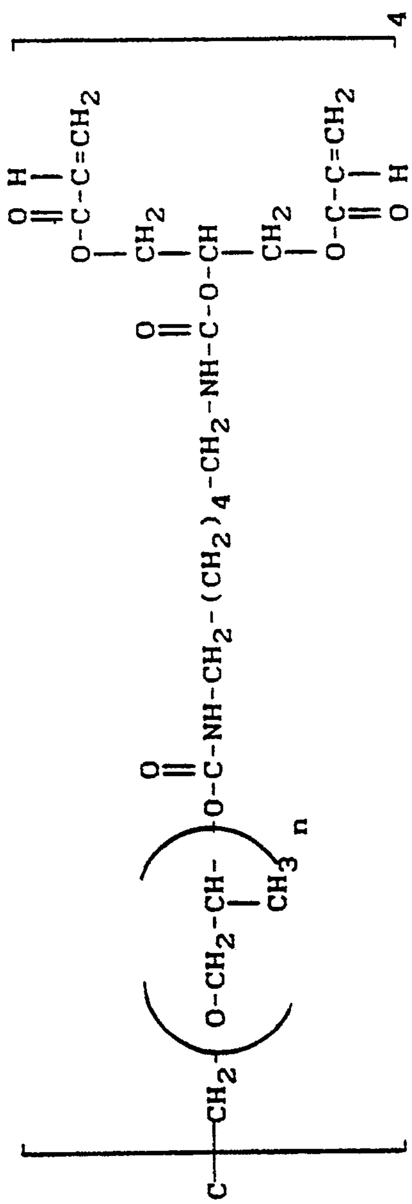

n = 1,225 (statistischer Mittelwert für 4 Ketten)

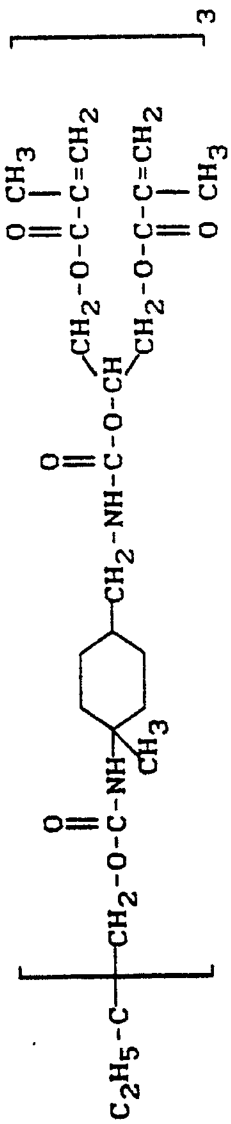

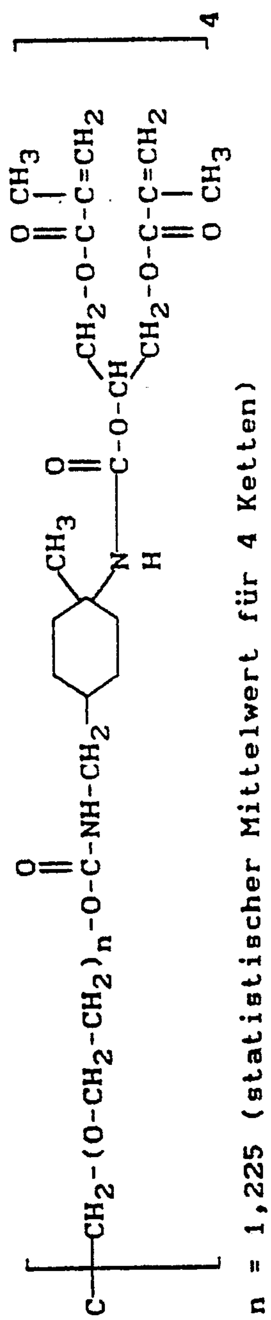

n = 1,225 (statistischer Mittelwert für 4 Ketten)

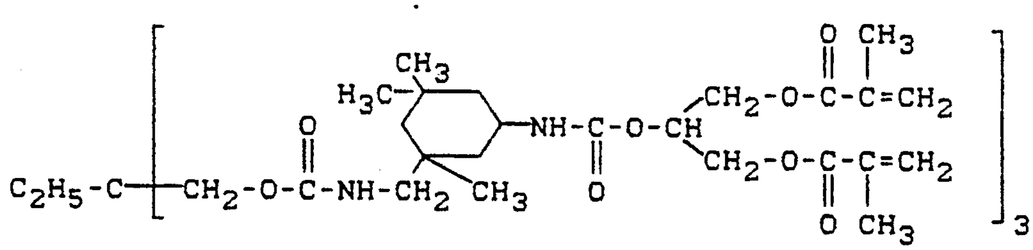

Besonders bevorzugt als Monomer wird das sogenannte Bis-GMA der Formel

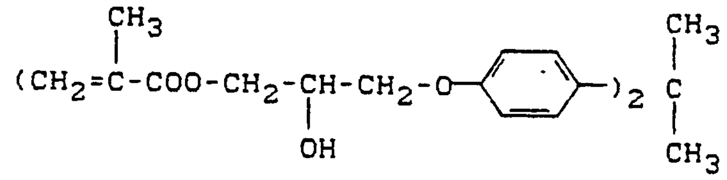

Selbstverständlich ist es möglich, Mischungen der verschiedenen (Meth)-Acrylsäureester, die Vernetzungen ausbilden können, einzusetzen. Beispielsweise seien Mischungen von 20 bis 70 Gew.-Teilen Bis-GMA und 30 bis 80 Gew.-Teilen Triethylenglykoldimethacrylat genannt.

Die erfindungsgemäßen Zubereitungen enthalten im allgemeinen 5 bis 80 Gew.-Teile, bevorzugt 10 bis 60 Gew.-Teile Carboxylverbindungen, bezogen auf die N-Formylpiperazinderivate.

Als weitere Komponente können die erfindungsgemäßen Zusammensetzungen Füllstoffe enthalten. Als Füllstoffe werden feine Pulver bevorzugt, die einen Teilchendurchmesser im Bereich von 0,1 bis 100 µm (gegebenenfalls auch in einer polydispersen Verteilung) haben. Füllstoffe können im Dentalbereich übliche Füllstoffe (R. S. Baratz, J. Biomat. Applications, Vol 1, 1987, S 316 ff) wie anorganische Gläser, Siliziumdioxid-, Aluminiumoxid-oder Quarzpulver sein.

Durch einen Anteil an Füllstoffen in den erfindungsgemäßen Zubereitungen entstehen Klebezemente, die besonders zur Befestigung von Brücken-, Kronen- und anderen Verblendmaterialen geeignet sind.

Der Anteil des Füllstoffs beträgt im allgemeinen 20 bis 80 Gew.-Teile, bevorzugt 40 bis 70 Gew.-Teile, bezogen auf die gesamte Zubereitung.

Die Adhäsivkomponenten gemäß dieser Erfindung können weiterhin bis zu 10 Gew.-Teilen üblicher Zusätze wie Stabilisatoren, Inhibitoren, Lichtschutzmittel, Farbstoffe, Pigmente oder Fluoreszenzstoffe enthalten.

Die erfindungsgemäßen Zubereitungen können hergestellt werden, indem man die Formylpiperazinggruppen enthaltenden (Meth)-acrylsäureester bzw. Methacrylsäureamide, Initiatoren und gegebenenfalls die anderen Komponenten durch kräftiges Rühren mischt.

Die Zubereitungen können auch lösungsmittelfrei vorliegen.

Die erfindungsgemäßen Zubereitungen können als Adhäsivkomponente zur Behandlung kollagenhaltiger Materialien verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5.

Diese enthält im allgemeinen Säuren mit einem $pK_s$-Wert kleiner als 5 und gegebenenfalls eine amphotere Aminoverbindung mit einem $pK_s$-Wert im Bereich von 9,0 bis 10,6 und einem $pK_B$-Wert im Bereich von 11,5 bis 12,5. Folgende Säuren können z.B. in der Konditionierflüssigkeit enthalten sein: Phosphorsäure, Salpetersäure, Brenztraubensäure, Zitronensäure, Oxalsäure, Ethylendiamintetraessigsäure, Essigsäure, Weinsäure, Äpfelsäure, Maleinsäure.

Als amphotere Aminoverbindungen seien vorzugsweise Verbindungen der Formel

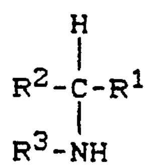

in der

$R^1$ für eine Carboxylgruppe steht,

$R^2$ Wasserstoff, gegebenenfalls durch Hydroxy, Thio, Methylthio, Carboxy, Amino, Phenyl, Hydroxy-phenyl oder die Gruppen

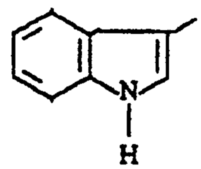 , 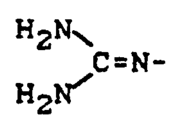 ,

substituierter Niederalkylrest,
$R^3$ Wasserstoff oder Phenyl bedeutet,
wobei die Reste $R^1$ und $R^3$ durch einen Propylrest verbunden sein können, oder
in der
$R^1$ für Wasserstoff steht,
$R^2$ für die Gruppe
$-A-NH_3X$ ,
in der
A für einen zweibindigen Alkylenrest mit 1 bis 6 Kohlenstoffatomen und
X für Halogen steht,
bedeutet und
$R^3$ Wasserstoff bedeutet,
genannt.

Beispielsweise seien die folgenden amphoteren Aminoverbindungen genannt: Glycin, Serin, Threonin, Cystein, Tyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiamin hydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid.

Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:

Bei einer Zahnreparatur zum Beispiel trägt man beispielsweise nach einer mechanischen Reinigung des kollagenhaltigen Zahnmaterials zuerst die Konditionierflüssigkeit mit etwas Watte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem kleinen Pinsel in einer dünnen Schicht auf und trocknet im Luftstrom. Nach der erfindungsgemäßen Behandlung trägt man die eigentliche Füllmasse, beispiels weise im Dentalbereich übliche Kunststoffüllungsmassen (K. Eichner, "Zahnärztliche Werkstoffe und ihre Verarbeitung", Bd. 2, S. 135 ff, Hüthig Verlag, 5. Aufl. 1985) auf.

In ähnlicher Weise können die erfindungsgemäßen Zubereitungen zur Befestigung von Kronen, Brücken und ähnlichen Hilfsmitteln verwendet werden.

Beispiel 1:

Synthese der neuen Formylpiperazingruppen enthaltenden (Meth)acrylsäureester am Beispiel des N-Formyl-N′-(metharyloyloxyethyl)piperazins

Vorstufe:

Zu einer gerührten Lösung aus 520,8 g (4,00 mol) N-2-Hydroxyethylpiperazin in 500 ml Methanol wurden 80 g (1,33 mol) Ameisensäuremethylester getropft und vier Stunden zum Rückfluß erhitzt. Nach Abziehen leicht flüchtiger Bestandteile wurde der erhaltene Rückstand bei 0,1 Torr rektifiziert, wobei zwischen 135 und 140 °C 169,6 g (81 % der Theorie) N-Formyl-N′-2-hydroxyethylpiperazin in Form einer farblosen Flüssigkeit erhalten wurden.

IR (Film): $\nu$ = 3400, 2930, 1680, 1660, 1440, 1400, 1275, 1218, 1142, 1047, 1008, 760 $cm^{-1}$
$^1H$-NMR ($CDCl_3$, 200 MHz): $\delta$ = 2.53

(m, 6H, 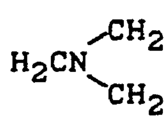 ),

3,26 (s, 1H, OH), 3,41, 3,56, (2t, J = 5 Hz, je 2H, CON-$CH_2$), 3,67 (t, J = 6 Hz, 2H, $OCH_2$), 8,03 (bs, 1H,

CHO)ppm.

Folgestufe:

Zu einer gerührten Lösung aus 160 g (1,01 mol) N-Formyl-N′-2-hydroxyethylpiperazin, 111 g (1,10 mol) Triethylamin und 100 mg 2,6-Di-tert.butyl-4-methylphenol in 300 ml Dichlormethan wurden zwischen -30 und -35 °C 105,5 g (1,01 mol) Methacrylsäurechlorid getropft und zwei Stunden bei gleicher Temperatur gerührt. Der ausgefallene Niederschlag wurde abfiltriert, das Filtrat mit Natriumchlorid-Lösung gewaschen, getrocknet und zu 166,8 g (73 % der Theorie) eines klaren Öles von N-Formyl-N′-(methacryloyloxyethyl)-piperazin eingeengt. Dieses ließ sich von einer Kieselgelsäule nach Abtrennung kleiner Mengen an Nebenprodukten mit Ether/n-Hexan (6:4) methanolisch in hoher Reinheit eluieren.

IR (Film): $\nu$ = 2940, 2800, 1725, 1675, 1442, 1400, 1318, 1294, 1194, 1160, 1010, 940 $cm^{-1}$

$^1H$-NMR ($CDCl_3$, 200 MHz): $\delta$ = 1,96 (bs, 3H, $CH_3$), 2,53

(m, 4H, 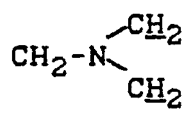 ),

2,72 (t, J = 6Hz, 2H, O-$CH_2CH_2$-N), 3,39, 3,57 (2m, je 2H, $H_2C$-N-CO), 4,29 (t, J = 6Hz,2H,$OCH_2$), 5,59, 6,10 (2bs, je 1H, vinyl.-H), 8,02 (bs, 1H, CHO)ppm.

Beispiel 2:

Synthese der neuen Formylpiperazingruppen enthaltenden (Meth)acrylsäureamide am Beispiel des N-Formyl-N′-(methacryloylaminoethyl)piperazins

Vorstufe:

Entsprechend Beispiel 1 wurden 774,3 g (6,00 mol) N-2-Aminoethylpiperazin in 300 ml Methanol mit 120,0 g (2,00 mol) Ameisensäuremethylester umgesetzt und bei 0,1 Torr zwischen 115 und 120 °C 151,6 g (48 % der Theorie) N-2-Aminoethyl-N′-formylpiperazin in Form einer gelblichen Flüssigkeit erhalten.

IR (Film): $\nu$ = 3300, 2930, 2800, 1668, 1441, 1396, 1272, 1203, 1130, 1013 $cm^{-1}$

$^1H$-NMR ($CDCl_3$, 200 MHz): $\delta$ = 1,64 (bs, 2H, $NH_2$), 2,47

m, 6H, 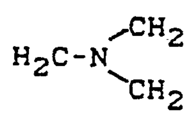 ),

2,81 (t, J = 6Hz, $CH_2NH_2$), 3,40, 3,48 (2t, J = 5Hz, je 2H, CON-$CH_2$), 8,02 (s, 1H, CHO)ppm.

Folgestufe:

117,9 g (0,75 mol) N-2-Aminoethyl-N′-formylpiperazin, 85,8 g (0,85 mol) Triethylamin und 100 mg 2,6-Di-tert.butyl-4-methylphenol wurden in 230 ml Dichlormethan mit 78,4 g (0,75 mol) Methacrylsäurechlorid wie in Beispiel 1 beschrieben umgesetzt und aufgearbeitet. 73,9 g (44 % d. Th.) N-Formyl-N′-(methacryloylaminoethyl)piperazin wurden auf diese Weise als farbloses Öl erhalten.

IR (Film): $\nu$ = 3350, 2950, 2820, 1675, 1665, 1618, 1532, 1442, 1221, 1152, 101, 925 $cm^{-1}$

$^1H$-NMR ($CDCl_3$, 200 MHz): $\delta$ = 1,98 (bs, 3H, $CH_3$), 2,50

(m, 8H, 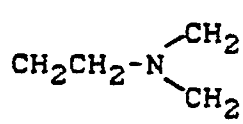 ),

3,42, 3,56 (2t, je 2H, $H_2C$-N-CO), 5,35, 5,70 (2 bs, je 1H, vinyl.-H), 6,52 (bs, 1H, NH), 8,03 (s, 1H, CHO)ppm.

Beispiele 3 bis 6: Herstellung der Zubereitungen (II)

Die erfindungsgemäßen Klebemittel werden durch intensives Vermischen der in folgenden Beispielen aufgeführten Bestandteile erzeugt.

Beispiel 3: 47 g Wasser
53 g N-Formyl-N′-(methacryloyloxyethyl)piperazin
159 mg Campherchinon

Beispiel 4: 36 g Wasser
48 g N-Formyl-N′-(methacryloyloxyethyl)piperazin
16 g 25 Gew.-%ige wäßrige Glutardialdehyd-Lösung
144 mg Campherchinon

Beispiel 5: 48 g Wasser
52 g N-Formyl-N′-(methacryloylaminoethyl)piperazin
144 mg Campherchinon

Beispiel 6: 37 g Wasser
48 g N-Formyl-N′-(methacryloylaminoethyl)piperazin
15 g 25 Gew.-%ige wäßrige Glutardialdehyd-Lösung
144 mg Campherchinon

Beispiel 7: (Anwendung)

Die Eignung der Klebemittel (II) entsprechend der Beispiele 3 bis 6 wird geprüft, indem die Bindungsfestigkeit der lichtaktivierten Kunststoffüllungsmasse auf Basis mehrfunktioneller Methacrylsäureester und Bariumaluminosilikat Lumifor® auf Dentin bestimmt wird, das nacheinander mit der Konditionierflüssigkeit (bestehend aus 81,2 g Wasser, 1,7 g Natriumhydroxid und 17 g Ethylendiamintetraessigsäure-Dinatrium-Dihydrat: 60 Sekunden Einwirkzeit, Wasserspülung, Lufttrocknung), dem Klebemittel (60 Sekunden Einwirkzeit, Lufttrocknung) und einem Verschlußmaterial auf Basis polyfunktioneller Methacrylsäureester (Bayer Resin L®) (Auftragen und im Luftstrom dünn verteilen) vorbehandelt worden ist.

Für den Test werden herausgezogene und im feuchten Zustand aufbewahrte Menschenzähne benutzt. Die Zähne werden durch Guß in Epoxidharz eingelagert; durch Nachschleifen wird eine glatte Dentinoberfläche erzeugt. Das anschließende Schleifen erfolgt mit Carbonpapier 1000.

Zur Herstellung eines Probekörpers zum Messen der Bindungsstärke wird eine zylindrische, gespaltete Teflonform auf die wie vorstehend beschrieben behandelte Dentinoberfläche gespannt (Scand. J. Dent. Res. 88, 348-351 (1980)). Als Füllmasse wird ein handelsübliches Kunststoffüllungsmaterial eingefüllt. Ein in einem Loch in einer Bohrerhalterung eingespannter Rundbohrer Nr. 016 wird an der Teflonform befestigt und von oben in die noch im Härteprozeß befindliche Materialschicht gepreßt.

Die gesamte Anordnung wird 10 Minuten lang bei Zimmertemperatur (25 °C) ungestört stehengelassen, wonach die Bohrerhalterung und die Teflonform abgenommen und die Probe unter Wasser mit einer Temperatur von 23 °C abgesetzt wird. Nach 15 Minuten wird die Probe mit dem Bohrer in eine Instron-Zugversuchsapparatur (Scand. J. Dent. Res. 88, 348-351 (1980)) montiert; mit einer Geschwindigkeit von 1 mm/Min. wird eine Zugfestigkeitsmessung durchgeführt. Die Zugfestigkeit errechnet sich aus der Division der beim Bruch der Füllung angelegten Belastung mit dem Querschnittsareal in der Bruchfläche des Probekörpers. Es wurden jeweils 5 Messungen an Probekörpern durchgeführt.

Die Ergebnisse sind in der folgenden Tabelle zusammengefaßt:

| Zubereitung nach Beispiel Nr. | Zugbindungsfestigkeit [N/mm$^2$] |
|---|---|
| 3 | 7 ± 2 |
| 4 | 12 ± 3 |
| 5 | 9 ± 2 |
| 6 | 9 ± 3 |

## Ansprüche

1. Formylpiperazingruppen enthaltende (Meth)acrylsäurederivate der Formel (I)

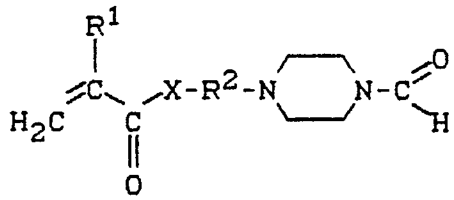

(I),

in der
$R^1$ Wasserstoff oder Methyl bedeutet,
$R^2$ ein zweiwertiger aliphatischer oder cycloaliphatischer Rest bedeutet und
X die Bedeutung von -O- oder -NH- hat.

2. Formylpiperazingruppen enthaltende (Meth)acrylsäurederivate nach Anspruch 1 worin
$R^2$ Ethylen bedeutet.

3. Formylpiperazingruppen enthaltende (Meth)acrylsäurederivate nach Anspruch 1, worin
$R^2$ Cyclohexylen bedeutet.

4. Zubereitungen, enthaltend Formylpiperazingruppen enthaltende (Meth)acrylsäurederivate gemäß den Ansprüchen 1 bis 3, und gegebenenfalls Initiatoren als Adhäsivkomponente zur Behandlung kollagenhaltiger Materialien.

5. Zubereitung gemäß Anspruch 4, dadurch gekennzeichnet, daß ein Coaktivator zugesetzt wird.

6. Zubereitung gemäß Anspruch 4, dadurch gekennzeichnet, daß als weitere Komponente (Meth)-acrylsäureester, die Vernetzungen ausbilden können, zugesetzt werden.

7. Verwendung von Zubereitungen enthaltend Formylpiperazingruppen enthaltende (Meth)-acrylsäurederivate der Formel (I),

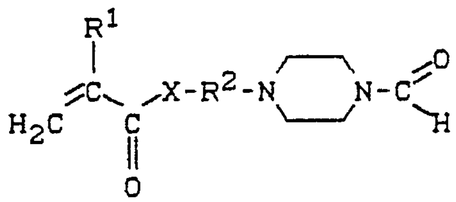

(I),

in der
$R^1$ Wasserstoff oder Methyl bedeutet,
$R^2$ ein zweiwertiger aliphatischer oder cycloaliphatischer Rest bedeutet und
X die Bedeutung von -O- oder -NH- hat
und gegebenenfalls Initiatoren, als Adhäsivkomponente zur Behandlung kollagenhaltiger Materialien.

8. Verwendung von Zubereitungen gemäß Anspruch 7 als Klebemittel zur Befestigung von Zahnreparaturmaterialien am Zahn.

9 Verwendung der Zubereitung nach Anspruch 7 als Klebemittel in Knochenzement.

10. N-Formyl-N′-aminoalkylpiperazine der Formel (V)

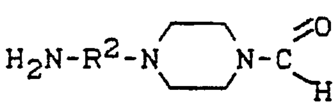

(V),

in der
$R^2$ ein zweiwertiger aliphatischer oder cycloaliphatischer Rest bedeutet.

Europäisches Patentamt

# EUROPÄISCHER RECHERCHENBERICHT

Nummer der Anmeldung

EP 90107142.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2 - 0 141 324 (BAYER AG) * Ansprüche * | 1,5,7, 8 | C 07 C 295/185<br>C 09 J 4/02<br>A 61 K 6/00<br>C 08 L 33/14<br>C 08 L 33/26 |
| A | US - A - 4 456 603 (YAMATSU et al.) * Beispiele 17,27,42 * | 1 | |
| A | DE - A1 - 2 550 547 (BASF) * Ansprüche; Seite 7, Zeile 6 * | 1,4 | |
| D,A | DE - A1 - 2 507 189 (BASF) * Ansprüche; Seite 5, Zeile 12 * | 1,4 | |
| D,A | DE - A1 - 3 703 080 (BAYER AG) * Ansprüche * | 1,4,9 | |
| A | GB - A - 742 450 (ABBOTT LABORATORIES) * Ansprüche * | 10 | |

RECHERCHIERTE SACHGEBIETE (Int Cl 5)

C 07 C 295/00
C 09 J
A 61 K 6/00
C 08 L 33/00

Der vorliegende Recherchenbericht wurde für alle Patentansprüche erstellt.

| Recherchenort | Abschlußdatum der Recherche | Prüfer |
|---|---|---|
| WIEN | 02-08-1990 | KÖRBER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument